Europäisches Patentamt

European Patent Office

Office européen des brevets

(11) Numéro de publication: **0 450 991 B1**

(12) # FASCICULE DE BREVET EUROPEEN

(45) Date de publication du fascicule du brevet: **18.10.95** (51) Int. Cl.6: **A61K 31/66**, A61K 31/685

(21) Numéro de dépôt: **91400304.1**

(22) Date de dépôt: **08.02.91**

Le dossier contient des informations techniques présentées postérieurement au dépôt de la demande et ne figurant pas dans le présent fascicule.

(54) **Utilisation de phosphatidylglycérol(diC 18:10)dans le traitement des maladies obstructives des voies aériennes.**

(30) Priorité: **20.02.90 FR 9001999**

(43) Date de publication de la demande:
**09.10.91 Bulletin 91/41**

(45) Mention de la délivrance du brevet:
**18.10.95 Bulletin 95/42**

(84) Etats contractants désignés:
**AT BE CH DE DK FR GB IT LI LU NL SE**

(56) Documents cités:
**EP-A- 0 446 017**
**WO-A-85/03638**
**WO-A-87/01586**
**WO-A-89/03220**
**US-A- 4 666 893**

**J.E.F REYNOLDS 'Martindale, the extra pharmacopoeia' 1982 , PHARMACEUTICAL PRESS , LONDON, GB pages 644-645**

(73) Titulaire: **SYNTHELABO**
**22, Avenue Galilée**
**F-92350 Le Plessis Robinson (FR)**

Titulaire: **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur: **Girod-Vaquez, Sophie, INSERM U 314**
**CHR Maison Blanche,**
**45, rue Cognacq Jay**
**F-51092 Reims Cédex (FR)**
Inventeur: **Puchelle, Edith, INSERM U 314**
**CHR Maison Blanche,**
**45, rue Cognacq Jay**
**F-51092 Reims Cédex (FR)**
Inventeur: **Galabert, Claude**
**Hôpital Renée Sabran**
**Giens,**
**F-83406 Hyeres (FR)**

Rank Xerox (UK) Business Services
(3.10/3.09/3.3.3)

REV. BRAS. CLIN. TER. vol. 9, no. 4, Avril 1980, BR pages 244 - 248 F.V.SUSO 'Tratamento da crise obstrutiva bronquica estado de mal asmatico'

PATENT ABSTRACTS OF JAPAN vol. 011, no. 381 (C-464)12 Décembre 1987

Inventeur: **Zahm, Jean-Marie, INSERM U 314 CHR Maison Blanche, 45, rue Cognacq Jay F-51092 Reims Cédex (FR)**
Inventeur: **Pierrot, Denis, INSERM U 314 CHR Maison Blanche, 45, rue Cognacq Jay F-51092 Reims Cédex (FR)**

(74) Mandataire: **Thouret-Lemaitre, Elisabeth et al SYNTHELABO, Service Brevets, B.P. 72 F-92352 Le Plessis-Robinson Cédex (FR)**

## Description

La présente invention a pour objet l'utilisation du phosphatidylglycérol (diC$_{18:0}$) pour la préparation d'un médicament utile dans le traitement de l'obstruction des voies aériennes.

On connaît d'après la demande de brevet WO-A-85/03638, des compositions constituées d'un mélange de phosphatidylcholine, de phosphatidyléthanolamine et de glycérides de jaune d'oeuf, utiles dans l'atténuation des symptômes de la mucoviscidose.

On connaît, d'autre part, d'après la demande de brevet WO-A-87/01586, des aérosols contenant des liposomes constitués de phospholipides, plus particulièrement de phosphatidylcholine, et d'un principe actif, permettant de libérer le principe actif dans les voies respiratoires et de contrôler sa libération.

Selon la présente invention, des propriétés particulières à un phospholipide, le phosphatidylglycérol (diC$_{18:0}$), ont été mises en évidence.

L'intégrité de l'épithélium de la muqueuse respiratoire dépend d'un équilibre entre les facteurs d'agression et les mécanismes de défense dont elle dispose. La première ligne de défense de l'arbre trachéo-bronchique est représentée par le mucus qui forme, à la surface des cellules épithéliales respiratoires, un film protecteur mobilisé en permanence par l'activité ciliaire.

Au cours des phénomènes inflammatoires bronchiques, aigus ou chroniques, l'hypersécrétion de mucus s'accompagne d'une modification de ses propriétés rhéologiques avec un accroissement de la viscosité et de l'adhérence. Ces anomalies se traduisent par une diminution de l'épuration muco-ciliaire et une stase du mucus avec obstruction bronchique. Le transport du mucus par la toux peut alors suppléer la déficience de la clairance muco-ciliaire. Il est donc très important de préserver l'efficacité de cette toux qui dépend non seulement des propriétés de viscosité et de visco-élasticité du mucus, mais qui peut être également largement influencée par les propriétés interfaciales entre le mucus et la muqueuse respiratoire.

Jusqu'à présent, les thérapeutiques visant à corriger l'hypersécrétion du mucùs et ses anomalies se sont toutes développées en partant du concept de base que le traitement de l'hypersécrétion devait avant tout porter sur la modification de la viscosité du mucus considérée comme seul facteur physique à l'origine de la stase du mucus et de l'obstruction bronchique. Ce concept a donné naissance à la mise au point de nombreuses molécules à activité mucolytique agissant *in vitro* ou *in vivo* et ayant pour but de normaliser l'hyperviscosité du mucus.

En fait, au cours des infections bronchiques aiguës et/ou chroniques, les sécrétions adhèrent fortement à la muqueuse et leur élimination par l'activité ciliaire, et plus encore par la toux, ne peut être que difficilement améliorée par la simple prescription d'une thérapeutique mucolytique. En effet, ces mucolytiques, qu'ils soient actifs in vivo ou in vitro, n'interviennent que sur la structure fibrillaire de la phase gel du mucus et n'ont aucune activité au niveau des propriétés tensio-actives du mucus.

Le mucus possède des constituants naturels biochimiques parmi lesquels figurent les phospholipides qui sont susceptibles de modifier les propriétés interfaciales mucus-muqueuse.

Il a été démontré dans de nombreuses études, en particulier dans le syndrome de détresse respiratoire aigu du nouveau-né, qu'un poumon immature pauvre en surfactant, c'est-à-dire en matériel tensio-actif, pouvait être rendu fonctionnel par la simple injection de surfactant artificiel ou naturel. Un grand nombre d'études ont également montré la présence de phospholipides, en particulier de phosphatidylcholine, dans le mucus respiratoire et les expectorations, aussi bien chez les sujets sains que chez les sujets atteints de maladies respiratoires avec hypersécrétion.

Les phospholipides, incluant les phospholipides à propriétés tensio-actives, se sont vus attribuer un rôle de stabilisant des petites voies aériennes. On leur a également attribué, en tant qu'agents tensio-actifs présents à l'interface phase sol-phase gel du mucus et grâce à leur propriétés hydrophobes, un rôle de régulation du contenu en eau de la phase sol. Par contre, malgré les hypothèses émises, aucun travail n'a montré jusqu'à maintenant, comment par leur présence à l'interface phase sol-phase gel du mucus, ces phospholipides pouvaient effectivement jouer un rôle protecteur de la muqueuse respiratoire et un rôle lubrifiant dans le transport de la phase gel sur la phase sol par le tapis ciliaire ou par le mécanisme de la toux.

En pathologie bronchique comme la mucoviscidose, des travaux antérieurs ont permis de montrer que dans les expectorations, certaines fractions lipidiques étaient augmentées et significativement corrélées à la viscosité (cholestérol, glycosphingolipides, sphingomyéline) et pourraient donc être considérées comme des facteurs "rigidifiants". A l'opposé, il a été montré que certaines fractions phospholipidiques, comme le phosphatidylglycérol, étaient au contraire négativement corrélées à la viscosité. Il apparaissait que le phosphatidylglycérol, bien que retrouvé en faible quantité dans les sécrétions bronchiques (10 % des phospholipides totaux), était susceptible de faciliter le transport du mucus par le mouvement des cils de la muqueuse respiratoire ou par le phénomène de la toux en modifiant les propriétés visco-élastiques du mucus (référence : Galabert et al, Clin. Chim. Acta, 1987, <u>164</u> : 139-149).

Les Demanderesses ont étudié le rôle des différentes fractions phospholipidiques, connues au niveau alvéolaire pour abaisser la tension de surface, dans les modifications des propriétés tensio-actives du mucus au niveau des voies aériennes. Les travaux ont débuté par une étude chez des patients atteints de mucoviscidose et de bronchopathies chroniques obstructives, chez lesquels ont été mesurés en parallèle le contenu en fractions phospholipidiques des expectorations et les propriétés de surface des fractions phospholipidiques. Le lien entre les données biochimiques et les propriétés physiques des expectorations étudiées a été analysé.

Le recueil des expectorations est fait en évitant toute contamination salivaire. Les patients sont répartis en deux groupes composés de dix-huit mucoviscidosiques et de seize hypersécrétants bronchitiques chroniques avec obstruction. Les lipides ont été séparés et identifiés par des techniques classiques ; pour cela, les expectorations sont immédiatement congelées, puis lyophilisées : l'extraction des lipides se fait à partir du matériel lyophilisé.

L'extraction quantitative des lipides est effectuée suivant la méthode de Bligh E.G. and Dyer W.J. : A rapid method of total lipid extraction and purification. Can. J. Biochem. Physiol., 1959, <u>37</u>, 911-917 : 100 mg de matériel lyophilisé sont réhydratés dans 1 ml d'eau distillée. Les lipides sont alors extraits par un mélange chloroforme/méthanol (1v/2v), puis dans un deuxième mélange chloroforme/méthanol (1v/1v). Après addition d'eau, la phase chloroformique est séparée. Trois extractions complémentaires par addition de 2,5 ml de chloroforme sont ensuite effectuées. Les différentes phases chloroformiques sont alors réunies, évaporées à sec sous azote et $P_2O_5$, puis pesées avant d'être remises en solution dans du chloroforme.

Les phospholipides totaux et les fractions phospholipidiques séparées par chromatographie en couche mince bidimensionnelle ont été dosés par la méthode de Rouser et al (Lipids, 1970, <u>5</u>, 494-496) dont le principe est le microdosage du phosphore après minéralisation des phospholipides par l'acide perchlorique et séparation par chromatographie en couche mince bidimensionnelle.

A partir du dosage des phospholipides dans ces sécrétions, il a été montré que les phospholipides totaux étaient significativement plus élevés dans la mucoviscidose que dans les bronchopathies chroniques obstructives (respectivement 4,40 % contre 2,96 % de poids sec du mucus (p<0.01)). Le pourcentage relatif de phosphatidylcholine et de phosphatidylglycérol est significativement plus faible (p<0,001) dans la mucoviscidose que dans les bronchopathies chroniques obstructives (Tableau I). Les pourcentages relatifs de sphingomyéline et de phosphatidylsérine + phosphatidylinositol sont signficativement plus élevés (p<0,001) dans la mucoviscidose que dans les bronchopathies chroniques obstructives (Tableau I).

## TABLEAU I

### pourcentage des principales fractions phospholipidiques par rapport aux phospholipides totaux

|  | PG | PC | SM | PS+PI |
|---|---|---|---|---|
| Mucoviscidose | 4,41 | 36,32 | 22,24 | 15,10 |
| Bronchopathies | 7,48 | 46,47 | 12,44 | 11,76 |

PC : phosphatidylcholine ; PG : phosphatidylglycérol ; SM : sphingomyéline ; PS+PI : phosphatidylsérine + phosphatidylinositol.

Dans le cas de la mucoviscidose, où les sécrétions sont très adhérentes et très visqueuses, on trouve moins de fractions tensio-actives (phosphatidylcholine, phosphatidylglycérol) et plus de fractions "rigidifiantes" (sphingomyéline) dans les expectorations que dans les autres bronchopathies.

Une étude physique de ces sécrétions a été effectuée en parallèle. La propriété physique choisie est la mouillabilité des sécrétions qui renseigne sur la capacité d'une sécrétion à mouiller une surface. La mouillabilité est évaluée par un paramètre physique appelé angle de contact. L'angle de contact peut être défini comme l'angle existant entre la tangente à une goutte de liquide déposée sur un support solide (à l'interface liquide-air) et la surface du solide (à l'interface solide-liquide) au point triple Tp.

L'angle de contact est dépendant du solide, de la nature du liquide et de l'interaction entre eux. Dans cette étude, nous avons étudié le comportement des sécrétions bronchiques lorsqu'elles sont mises en contact avec un support qui simule la muqueuse bronchique. L'angle de contact est un index de mouillabilité qui renseigne sur les propriétés tensio-actives du liquide pour un solide donné : plus l'angle de contact est grand, moins le liquide s'étale et moins il est mouillant.

Une technique de mesure automatique de l'angle de contact basée sur le traitement d'image associé à un traitement informatique a été développée. Dans les mesures de mouillabilité des sécrétions, il a été choisi de mesurer l'angle de contact d'une goutte calibrée (5 $\mu$l) de sécrétion mise en contact avec un support de verre parfaitement propre, chargé électronégativement et placé dans une enceinte saturée en vapeur d'eau pour éviter les phénomènes de deshydratation. (référence : S. Girod et al., I.T.B.M., 1988, 9, 402-412.

Le phosphatidylglycérol apparaît comme un phospholipide important contrôlant la mouillabilité des sécrétions bronchiques et susceptibles d'influencer leur transport et leur comportement lorsqu'elles sont en contact avec la muqueuse. Les Demanderesses ont donc étudié l'influence de différents phospholipides sur les propriétés de mouillabilité du mucus respiratoire et sur sa capacité à être mobilisé et transporté par le mécanisme de la toux. Une expérimentation visant à étudier l'effet in vitro de différentes fractions phospholipidiques sur la mouillabilité et le transport par la toux de mucus considérés comme "normaux" a donc été mise en oeuvre.

Les mucus sont prélevés sur la muqueuse du palais de grenouille considérée comme un bon modèle représentatif de la muqueuse respiratoire humaine. La mouillabilité de ces mucus est mesurée comme il a été décrit précédemment. La clairance par la toux de ces mucus a été mesurée à l'aide d'une machine à tousser. Le principe de cette machine est le suivant : le support sur lequel la goutte de mucus est déposée est placé comme plancher d'un tube en plexiglas qui simule la trachée. Ce tube en plexiglas d'une section rectangulaire (20 mm x 10 mm) est connecté à une enceinte fermée par une électrovanne. La pression dans l'enceinte est amenée à 0,6 bar, puis l'électrovanne est ouverte et le débit d'air que subit la goutte est de 6 l/s. On mesure le déplacement de la goutte sur le support sous l'influence du courant d'air ainsi provoqué qui simule le mécanisme de la toux.

Les supports utilisés sont soit des lames de verre simples, soit des lames de verre recouvertes d'une monocouche de phospholipides grâce à une balance à film de Langmuir : 10 mg de chaque fraction phospholipidique sont dissous dans un mélange chloroforme/méthanol (9:1) et 16 $\mu$l de cette solution sont déposés dans la cuve de la balance à film remplie d'eau tridistillée. Une barrière de téflon comprime le film phospholipidique jusqu'à la pression de collapse propre à chaque phospholipide, où toutes les molécules de phospholipide sont en contact sans trou dans la monocouche. Le support de verre préalablement immergé dans la cuve est ensuite retiré à une vitesse constante et, parallèlement, la barrière de téflon avance de façon à maintenir également constante la tension de surface du film ainsi prélevé ; le support de verre ainsi obtenu présente en surface une monocouche orientée de phospholipides accrochés par leur tête polaire au verre et exposant à l'air leurs groupements apolaires (en contact avec le mucus).

Les phospholipides choisis pour cette étude sont le phosphatidylglycérol (diC$_{18:1}$) (Sigma 9399) et le phosphatidylglycérol (diC$_{18:0}$) (Sigma 9524) : les deux fractions ont donc la même tête polaire, la même longueur de chaînes apolaires mais des degrés de saturation différents. On garde comme témoins des lames de verre non recouvertes de phospholipides.

Il ressort que le phosphatidylglycérol (diC$_{18:0}$) améliore significativement la clairance par la toux du mucus de grenouille et diminue significativement sa mouillabilité, en comparaison avec les lames de verre non recouvertes ($p < 0,001$ et $p < 0,01$) ou même avec les lames de verre recouvertes de phosphatidylglycérol (diC$_{18:1}$) ($p < 0,001$ et $p < 0,01$). Le phosphatidylglycérol (diC$_{18:0}$) semble être la fraction phospholipidique qui en diminuant les propriétés d'adhésion du mucus en améliore sa clairance par la toux (la fraction la plus lubrifiante).

La phosphatidylcholine sous forme de dipalmitoyl-phosphatidyl-choline (diC$_{16:0}$) a également été étudiée sur la mouillabilité et la capacité de transport par la toux.

Compte tenu de ces études, les Demanderesses revendiquent l'utilisation du phosphatidylglycérol (diC$_{18:0}$) seul ou en association avec des excipients appropriés, pour la préparation d'un médicament utile dans le traitement de l'obstruction des voies aériennes.

Le phosphatidylglycérol (diC$_{18:0}$) peut être utilisé pour la préparation de médicaments présentés sous la forme de suspensions micellaires ou sous la forme de suspensions de liposomes, et administrés par voie orale ou par voie aérienne, par exemple par inhalation (sous la forme d'aérosols) ou par instillation.

Il peut également être associé à des agents thérapeutiques connus par leur activité dans le domaine des maladies bronchopulmonaires et susceptibles d'avoir une activité propre sur la muqueuse bronchique.

En particulier, il est possible d'associer au phosphatidylglycérol (diC$_{18:0}$) sous forme de suspensions micellaires (ou d'émulsions) ou sous forme de suspensions de liposomes, en instillations ou en aérosols, les composés suivants :

- des agents protecteurs contre les radicaux libres tels que les dérivés de la cystéine ou la superoxyde dismutase,
- des substances antibactériennes telles que le lysozyme ou l'asialo-GM1-ganglioside
- des facteurs de croissance tels que insuline, dérivés de l'acide rétinoïque, epithelium growth factor (ou EGF) et platelet-derived-growth factor (ou PDGF),
- des facteurs de réparation tels que la glycyl-L-histidyl-L-lysine et d'autres peptides purifiés à partir de cellules épithéliales ou mésenchymateuses,
- des agents d'hydratation du mucus tels que l'ATP.

**Revendications**

1. Utilisation du phosphatidylglycérol (diC$_{18:0}$) pour la préparation d'un médicament utile dans le traitement de l'obstruction des voies aériennes.

2. Utilisation selon la revendication 1, pour la préparation d'un médicament présenté sous forme de suspension micellaire.

3. Utilisation selon la revendication 1, pour la préparation d'un médicament présenté sous forme de suspension de liposomes.

4. Utilisation selon la revendications 1, caractérisée en ce que le phosphatidylglycérol (diC$_{18:0}$) est associé à un agent ayant une activité propre sur la muqueuse bronchique.

5. Utilisation selon la revendication 4, caractérisée en ce que l'agent ayant une activité propre sur la muqueuse bronchique est choisi parmi les composés suivants :
   - agents protecteurs contre les radicaux libres tels que les dérivés de la cystéine ou la superoxyde dismutase,
   - substances antibactériennes telles que le lysozyme ou l'asialo-GM1-ganglioside,
   - facteurs de croissance tels que insuline, dérivés de l'acide rétinoïque, epithelium growth factor (ou EGF) et platelet-derived-growth factor (ou PDGF),
   - facteurs de réparation tels que la glycyl-L-histidyl-L-lysine et d'autres peptides purifiés à partir de cellules épithéliales ou mésenchymateuses,
   - agents d'hydratation du mucus tels que l'ATP.

**Claims**

1. Use of phosphatidylglycerol (diC$_{18:0}$) for the preparation of a medicinal product which is useful in the treatment of obstruction of the airways.

2. Use according to Claim 1, for the preparation of a medicinal product provided in the form of a micellar suspension.

3. Use according to Claim 1, for the preparation of a medicinal product provided in the form of a liposome suspension.

4. Use according to Claim 1, characterized in that the phosphatidylglycerol (diC$_{18:0}$) is combined with an agent which has a specific activity on the bronchial mucosa.

5. Use according to Claim 4, characterized in that the agent which has a specific activity on the bronchial mucosa is chosen from among the following compounds:
   - protective agents against free radicals such as cysteine derivatives or superoxide dismutase,
   - antibacterial substances such as lysozyme or asialo-GM1-ganglioside
   - growth factors such as insulin, retinoic acid derivatives, epithelium growth factor (or EGF) and platelet derived growth factor (or PDGF),

- repair factors such as glycyl-L-histidyl-L-lysine and other peptides purified from epithelial or mesenchymatous cells,
- mucus-hydrating agents such as ATP.

**Patentansprüche**

1. Verwendung von Phosphatidylglycerin (diC$_{18:0}$) für die Herstellung eines Arzneimittels zur Behandlung von Verschlüssen der Atemwege.

2. Verwendung nach Anspruch 1 für die Herstellung eines Arzneimittels in Form einer Micellensuspension.

3. Verwendung nach Anspruch 1 für die Herstellung eines Arzneimittels in Form einer Suspension von Liposomen.

4. Verwendung nach Anspruch 1, **dadurch gekennzeichnet,** daß das Phosphatidylglycerin (diC$_{18:0}$) mit einem Mittel kombiniert ist, das eine eigene Wirkung auf die Bronchienschleimhaut ausübt.

5. Verwendung nach Anspruch 4, **dadurch gekennzeichnet,** daß das Mittel mit eigener Wirkung auf die Bronchienschleimhaut aus den folgenden Verbindungen ausgewählt ist:
   - Mittel, die gegen freie Radikale schützen, wie Cysteinderivate oder Superoxid-Dismutase,
   - antibakterielle Substanzen, wie Lysozym oder Asialo-GM1-gangliosid,
   - Wachstumsfaktoren, wie Insulin, Derivate der Retinoesäure, Epitheliumwachstumsfaktor(oder EGF) und plättchenabgeleitete Wachstumsfaktor (oder PDGF),
   - Verteilungsfaktoren, wie Glycyl-L-histidyl-L-lysin und andere gereinigte Peptide ausgehend von Epithelial- oder Mesenchymzellen,
   - Schleimhaut befeuchtende Mittel, wie ATP.